# FASCICULE DE BREVET EUROPEEN

(11) **EP 0 418 112 B1**
(45) Date de publication et mention de la délivrance du brevet: **02.08.1995**
(21) Numéro de dépôt: 90402404.9
(22) Date de dépôt: 31.08.1990
(51) Int. Cl.: G01N 33/24

(54) **Dispositif et méthode d'évaluation de l'aptitude qu'a un corps à expulser un produit et leur application à un échantillon de roche**
Vorrichtung und Verfahren zur Schätzung von der Fähigkeit eines Körpers um ein Produkt austreiben zu können und ihre Anwendung auf eine Gesteinsprobe
Apparatus and method for evaluating the aptitude of a body to expel a product and their application on a rock sample

(30) Priorité: 15.09.1989 FR 8912150
(43) Date de publication de la demande: 20.03.1991
(73) Titulaire: INSTITUT FRANCAIS DU PETROLE, 92506 Rueil-Malmaison Cédex (FR)
(72) Inventeur: Lafargue, Eric, F-75013 Paris (FR); Espitalie, Jean, F-78110 Le Vesinet (FR); Lesage, Thierry, F-78250 Tessancourt sur Aubette (FR)

(56) Documents cités:
- WO-A-88/00694
- DE-U- 8 707 159
- FR-A- 2 592 488
- US-A- 4 233 840
- US-A- 4 572 009

## Description

La présente invention concerne l'évaluation de l'aptitude qu'a un corps contenant au moins un produit à expulser celui-ci. Elle s'applique particulièrement bien aux roches renfermant des hydrocarbures.

La caractérisation des roches-mères repose actuellement essentiellement sur la détermination de la quantité et de la qualité des hydrocarbures qu'elles sont susceptibles de générer lors de leur enfouissement. Une telle caractérisation est insuffisante, car elle ne prend pas en compte la capacité que présentent de telles roches à expulser ou non les produits générés. Il est pourtant évident que dans un bassin sédimentaire, la présence d'une accumulation d'huile (ou de gaz) est fortement conditionnée par la possibilité qu'ont à l'origine les hydrocarbures de quitter ou non la roche-mère où ils ont été générés.

Le document FR-A-2.592.488 permet de caractériser la quasi-totalité de la matière organique extractible d'une roche sédimentaire, dans des conditions de pression et de température supercritiques qui peuvent se révéler contraignantes.

L'aptitude à expulser les hydrocarbures produits apparait donc comme étant un paramètre essentiel dans la détermination des bonnes roches-mères qui expulseront facilement les hydrocarbures qu'elles ont générés, par opposition aux mauvaises roches-mères qui n'expulseront que peu ou pas d'hydrocarbures. Il n'existe pas à l'heure actuelle de moyen permettant de définir ce paramètre. La présente invention propose une méthode et un dispositif permettant notamment de classer les roches en fonction de leur aptitude à expulser des hydrocarbures dans les conditions d'un test de migration réalisé en laboratoire sur des échantillons de roche.

Ainsi, d'une manière plus générale, la présente invention concerne une méthode permettant l'évaluation de l'aptitude qu'a un corps contenant un produit à expulser celui-ci. Cette méthode comporte les étapes suivantes :
- on place ledit corps et un réservoir de manière adjacente,
- on réalise un gradient de pression entre le réservoir et ledit corps, ce gradient provoquant l'expulsion vers ledit réservoir d'au moins une partie dudit produit, et
- on effectue, par exemple, une analyse qualitative et/ou quantitative du produit à expulser et on détermine un critère de migration lié au rapport de la quantité du produit expulsé à la quantité du corps initialement traité.

On pourra effectuer l'analyse de la partie du produit récupérée dans ledit réservoir par un balayage en continu de ce réservoir.

La méthode selon l'invention pourra comporter une étape de chauffage dudit corps et/ou une mise en pression de celui-ci.

La méthode pourra permettre d'évaluer la quantité d'hydrocarbure contenue dans un échantillon de roche. Elle pourra de même être appliquée à l'évaluation de la quantité de produit autre que les hydrocarbures contenus par ladite roche notamment à l'eau, au monoxyde de carbone, au dioxyde de carbone ou au dioxyde de soufre.

La présente invention concerne également un dispositif permettant l'évaluation de l'aptitude qu'a un corps contenant au moins un produit à expulser celui-ci. Ce dispositif comporte une enceinte haute pression comportant elle-même un milieu de confinement, et des moyens de génération d'une pression de confinement. Il comporte, en outre, un ensemble, ou éprouvette, hermétiquement isolé du milieu de confinement, cet ensemble comportant un échantillon du corps et un réservoir.

Le dispositif pourra comporter des moyens de contrôle du gradient de pression existant entre l'échantillon et le réservoir.

Le dispositif pourra comporter des moyens de balayage dudit réservoir.

Les moyens de balayage pourront comporter des moyens de détection et/ou d'analyse des fluides recueillis par balayage dans le réservoir.

Le dispositif selon l'invention pourra comporter des moyens de régulation en température des fluides de balayage.

Le dispositif selon l'invention peut comporter principalement une enceinte, ou cellule haute pression, dans laquelle est placé un assemblage constitué par la superposition ou la juxtaposition d'un échantillon de roche et d'un réservoir au sein d'un ensemble isolé hermétiquement du milieu de confinement, de préférence par une jaquette métallique et deux bouchons en acier. Dans la suite de ce texte, on pourra également désigner cet ensemble par le terme d'éprouvette. Sous l'effet de la pression de confinement, les fluides présents dans l'échantillon de roche sont expulsés vers le réservoir où éventuellement un système de balayage peut assurer leur transport vers des détecteurs spécifiques, permettant ainsi une analyse en continu des produits expulsés.

L'originalité de la présente invention réside, notamment, dans la juxtaposition de l'échantillon de roche et du réservoir dans un ensemble hermétique relativement au milieu de confinement. De plus, pour certains modes particuliers de réalisation, elle réside en outre dans l'analyse en continu des produits expulsés grâce au dispositif de balayage. Dans ce dernier cas, il est donc possible d'étudier la cinétique de l'expulsion, ce qui ne serait pas possible si on avait à démonter la cellule après chaque expérience.

La présente invention sera mieux comprise et ses avantages apparaitront plus clairement à la description qui suit d'un exemple particulier, nullement limitatif, appliqué au cas des roches pétrolifères, illustré par les figures ci-annexées.
- la figure 1 illustre dans son ensemble le dispositif selon l'invention,
- la figure 2 montre un abaque permettant le classement des roches, et
- la figure 3 représente de manière schématique la manière dont les prssions s'appliquent sur l'échantillon de roche et sur le réservoir.

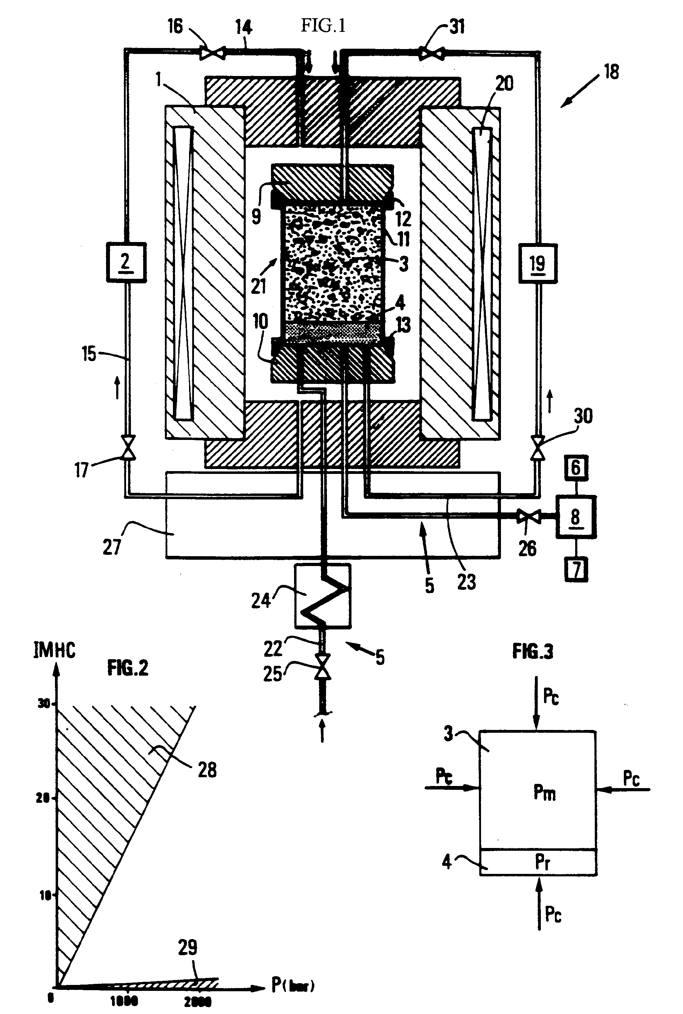

L'appareil illustré à la figure 1 comprend :
- un corps d'enceinte 1 haute pression, équipé d'un four 20, permettant de travailler à des pressions généralement comprises entre 1 et 4.000 bar et des températures généralement comprises entre 20 et 400°C, le four pouvant être intégré ou non au corps de l'enceinte,
- des moyens de génération de pression de confinement 2, et
- un ensemble 21 hermétiquement isolé du milieu de confinement lors des essais, cet ensemble ou éprouvette recevant l'échantillon à tester et un réservoir.

Le dispositif selon l'invention peut également comporter :
- des moyens de contrôle du gradient de pression 19 entre l'échantillon de roche 3 et le réservoir 4,
- des moyens de balayage des produits expulsés 5 dans le réservoir 4, et/ou
- des détecteurs spécifiques aux hydrocarbures 6, et éventuellement aux CO₂, CO ET H₂O, 7, reliés à une boucle d'échantillonnage 8.

L'éprouvette 21 contient l'échantillon de roche 3 et le réservoir 4 qui sont maintenus juxtaposés et isolés du milieu de confinement par l'intermédiaire de deux bouchons métalliques 9 et 10 et d'une jaquette métallique 11 sertie sur les précédents à l'aide de deux bagues métalliques 12 et 13, ayant éventuellement une section droite en forme de coin.

Les moyens de génération d'une pression de confinement 2 sont reliés à l'enceinte par des canalisations 14 et 15 qui comportent des vannes 16 et 17. Ces vannes permettent d'isoler le milieu de confinement des moyens 2 de génération de pression de confinement et éventuellement la vidange de l'enceinte de confinement lors de l'arrêt des moyens 2 de génération de la pression de confinement.

La référence 18 désigne dans leur ensemble les moyens servant à controler et éventuellement à créer le gradient de pression au sein de l'éprouvette 21. Ces moyens peuvent comporter une pompe et éventuellement des moyens de vannage 30, 31, notamment pour isoler l'espace interne de l'éprouvette de la pompe 19.

Les moyens 18 permettant de controler le gradient de pression entre la roche et le réservoir comportent de manière classique un capteur différentiel de pression relié à un système de régulation de la pression commandant une pompe pneumatique. Ces moyens 18 de contrôle du gradient sont désignés hors les canalisations et les moyens de vannage par la référence 19. Ces moyens 18 sont différents des moyens de génération de la pression de confinement.

Les moyens de génération de la pression de confinement peuvent comprendre une ou des pompes pneumatiques tout-à-fait classiques associées à des systèmes de régulation reliés à un capteur de pression mesurant la pression régnant dans l'enceinte de confinement.

Les moyens de balayage 5 peuvent comporter une source de fluide de balayage non représentée et un circuit d'entrée 22 amenant le fluide de balayage fourni par cette source au réservoir 4, à travers le bouchon 10 et un circuit de sortie 23 menant le fluide de balayage vers la boucle d'échantillonnage 8, après que celui-ci ait traversé le réservoir 4.

Le circuit d'entrée 22 peut comporter des moyens de chauffage permettant de réchauffer le fluide de balayage.

Le circuit de balayage comporte des vannes 25 et 26 permettant d'interrompre la circulation du fluide de balayage.

Pour éviter la condensation dans la canalisation du circuit de sortie 23 des moyens de balayage, celle-ci pourra cheminer au moins sur une grande partie de sa longueur dans un ensemble thermostaté 27. Les autres canalisations pourront également cheminer en partie dans lesdits moyens thermostatés, comme cela est représenté à la figure 1

Le dispositif selon l'invention permet de réaliser des tests d'expulsion sur des échantillons de roches naturelles, carottes ou plugs. Le diamètre de ces échantillons pourra être compris entre 20 et 50 mm pour une longueur comprise aussi entre 20 et 55 mm. Les roches utilisées peuvent être immature (c'es-à-dire n'ayant pas encore généré d'hydrocarbures lors de leur enfouissement) ou matures.

Dans le premier cas, on effectuera préalablement au test d'expulsion, la génération d'hydrocarbures dans les conditions de pression et de température appropriées dans l'enceinte haute pression par exemple, à une température comprise par exemple entre 300 et 400°C, et une pression voisine de 1.000 bar pendant 48 à 72 heures. Les produits expulsés dans ces conditions de température dans le réservoir durant cette phase peuvent être exploités en vue d'évaluer l'aptitude de migration des hydrocarbures dans ledit échantillon, ainsi que la qualité de l'opération de maturation artificielle Toutefois, il est préférable pour une exploitation aisée des informations de les éliminer du réservoir, par exemple, soit grâce au système de balayage, soit en changeant de réservoir. Ceci s'obtient en ouvrant l'éprouvette et en fabriquant une nouvelle contenant la roche artificiellement mature et un nouveau réservoir. On peut alors entreprendre le test d'expulsion proprement dit sur la roche artificiellement mature.

Pour une roche mature, on ne fait que le test d'expulsion, étant donné qu'elle a déjà généré naturellement des hydrocarbures au cours de son enfouissement.

Le test d'expulsion consiste à étudier la quantité et le type de produits expulsés dans le réservoir au cours du temps dans les conditions de pression et de température du test. Le réservoir pourra être une roche naturelle (grès, carbonates...), ou un matériau poreux quelconque (fritte métallique, céramique...). Son épaisseur pourra varier entre 2 et 20 mm. Le réservoir peut être vide à l'origine, ou contenir de l'eau. Dans le mode de réalisation décrit à la figure 1, les produits, une fois expulsés dans le réservoir, sont acheminés vers les différents détecteurs 6 et 7 par l'intermédiaire d'un système de balayage faisant intervenir un fluide chauffé (gaz inertes, eau avec ou sans tensio-actifs, CO₂, solvants organiques). L'ensemble est chauffé grâce à l'ensemble thermostaté 27, afin d'éviter l'apparition de points froids sur le circuit de balayage de sortie 23 où pourraient se condenser les produits expulsés, faussant ainsi les analyses quantitatives. A la sortie de la boucle d'échantillonnage 8, les hydrocarbures, l'eau, le CO, le CO₂ et l'H₂S sont analysés séparément.

Le dispositif selon l'invention est d'une grande souplesse et permet la réalisation de tests d'expulsion dans différentes configuration : pression de confinement seule ou avec en plus un gradient de pression imposé entre la roche et le réservoir. En cas de besoin, le système de balayage peut aussi être interrompu si on ne souhaite récupérer les produits expulsés qu'en fin d'expérience et non plus en continu.

Les gammes de température et de pression peuvent être étendues de 20 à 400°C et de 1 à 4.000 bar). Les pressions et les températures peuvent être enregistrées automatiquement et peuvent être programmées en fonction du temps.

Comme cela a été dit, le dispositif expérimental permet de déterminer l'aptitude d'une roche à expulser notamment les hydrocarbures, l'eau, le CO, le CO₂ et l'H₂S qu'elle a pu générer lors de son enfouissement. Grâce notamment à la définition d'un Index de Migration (IMHC), il est possible, selon la présente invention, de classer les roches-mères en roches-mères très bonnes, c'est-à-dire capables d'expulser facilement les hydrocarbures formés, par opposition aux mauvaises roches-mères, incapables d'expulser de façon notable les hydrocarbures qui y ont été générés.

On peut définir IMHC par exemple de la manière suivante :
IMHC = Q HC expulsés (mg) / 100 g roche initiale

L'abaque présenté à la figure 2 a été établi à partir d'expériences sur différentes roches et on a choisi 72 heures comme durée moyenne des expériences, en abscisse est présenté la pression de confinement en bar et en ordonnée le IMHC. La zone 28 correspond à de bonnes roches-mères, c'est-à-dire des roches expulsant facilement l'hydrocarbure qu'elles contiennent. A l'inverse, la zone 29 correspond aux mauvaises roches-mères. Il est bien entendu possible de donner des abaques similaires pour d'autres durées d'expériences.

Parallèlement à la définition de l'Index de Migration des hydrocarbures, il est possible de définir des index similaires pour les autres produits expulsés :
- Imeau = Qeau (mg) / 100 g roche initiale
- IMCO = Qeau (mg) / 100 g roche initiale
- IMCO₂ = QCO₂ (mg) / 100 g roche initiale
- IMH₂S = SH₂S (mg) / 100 g roche initiale

Enfin, la cinétique de l'expulsion déterminée à partir de l'analyse en continu des produits expulsés permet aussi de caractériser l'aptitude des roches testées à expulser les produits formés en fonction du temps, lors de leur enfouissement dans les basses couches sédimentaires.

La figure 3 illustre schématiquement les pressions auxquelles sont soumis l'échantillon 3 et le réservoir 4.

Sur cette figure, PC représente la pression de confinement qui s'applique au squelette de l'échantillon de roche 3 et à celui du réservoir 4. Cette pression PC correspond sensiblement à celle qui règne dans l'enceinte, du moins lorsque l'effort de résistance de la jaquette métallique est négligeable par rapport aux efforts créés par la pression de confinement.

Pm désigne la pression au sein des pores de la roche-mère qui est également appelée pression dans la roche-mère et Pr au sein des pores du réservoir ou pression dans le réservoir.

Le gradient de pression correspond à la différence : Pm - Pr. C'est ce gradient qui permet d'expulser les fluides hors de l'échantillon de roche 3.

Comme cela a été dit, ce gradient peut être contrôlé par les moyens 19, ou peut être généré simplement du fait de la pression de confinement et des différences de caractéristiques mécaniques entre la roche-mère 3 et le réservoir 4, celui-ci étant le plus résistant.

Au début de l'essai, le réservoir peut être vide ou plein d'un fluide tel de l'eau. Dans ce dernier cas, on imposera de préférence une pression de flux Pr dans le réservoir, telle que le gradient ΔP = Pm - Pr soit plus proche du gradient de pression naturel, généralement inférieur à 1.000 bar.

Un autre avantage de ce dernier cas est de permettre éventuellement le contrôle du gradient de pression lors de la génération artificielle des fluides dans les roches-mères immatures à des températures élevées (300 à 400°C) qui engendre des pressions de fluide élevées.

Le réservoir peut être une roche naturelle (grès, carbonate poreux, roche volcanique...), un matériel poreux de synthèse (fritte métallique, céramique...).

Le rapport porosité du réservoir / porosité de la roche-mère testée de préférence, ne doit pas être inférieur à 4 au début de l'expérience.

La jaquette pourra être réalisée en cuivre.

## Revendications

1. Méthode permettant l'évaluation de l'aptitude qu'a un corps contenant un produit à expulser celui-ci, caractérisée en ce qu'elle comporte les étapes suivantes :
- on place ledit corps et un réservoir de manière adjacente,
- on réalise un gradient de pression entre le réservoir et ledit corps, ce gradient provoquant l'expulsion vers ledit réservoir d'au moins une partie dudit produit, et
- on effectue une analyse qualitative et/ou quantitative dudit produit à expulser et on détermine un critère de migration lié au rapport de la quantité du produit expulsé à la quantité dudit corps initialement traité.

2. Méthode selon la revendication 1, caractérisée en ce que l'on effectue l'analyse de la partie du produit récupéré dans ledit réservoir par un balayage en continu dudit réservoir.

3. Méthode selon l'une des revendications 1 à 2, caractérisée en ce qu'elle comporte une étape de mise en pression dudit corps accompagnée éventuellement d'une opération de chauffage dudit corps.

4. Méthode selon l'une des revendications 1 à 3, caractérisée en ce que l'on évalue la quantité d'hydrocarbure contenue dans un échantillon de roche.

5. Méthode selon la revendication 4, caractérisée en ce que l'on évalue la quantité de produit autre que les hydrocarbures contenus par ladite roche notamment à l'eau, le monoxyde de carbone, le dioxyde de carbone ou au dioxyde de soufre.

6. Dispositif permettant l'évaluation de l'aptitude qu'a un corps contenant au moins un produit à expulser celui-ci, ledit dispositif comportant une enceinte haute pression (1) comportant elle-même un milieu de confinement, et des moyens de génération de pression de confinement (2), caractérisé en ce qu'il comporte un ensemble (21) ou éprouvette hermétiquement isolée du milieu de confinement, ledit ensemble comportant un échantillon dudit corps et un réservoir.

7. Dispositif selon la revendication 6, caractérisé en ce qu'il comporte des moyens de contrôle du gradient de pression (18) existant entre ledit échantillon et ledit réservoir.

8. Dispositif selon l'une des revendications 6 ou 7, caractérisé en ce que ledit dispositif comporte des moyens de balayage (5) dudit réservoir.

9. Dispositif selon la revendication 8, caractérisé en ce que lesdits moyens de balayage comportent des moyens de détection et/ou d'analyses (8, 6, 7) des fluides recueillis par balayage dans ledit réservoir.

10. Dispositif selon l'une des revendications 8 ou 9, caractérisé en ce qu'il comporte des moyens de régulation en température (24, 27) des fluides de balayage.

## Patentansprüche

1. Verfahren zur Bestimmung der Eignung, die ein Körper aufweist, welcher ein aus diesem auszutreibendes Produkt enthält, dadurch gekennzeichnet, daß es die folgenden Schritte umfaßt:
- man ordnet den Körper und einen Behälter benachbart zueinander an,
- man realisiert einen Druckgradienten zwischen dem Behälter und dem Körper, wobei dieser Gradient die Austreibung von wenigstens einem Teil des Produkts hin zu dem Behälter hervorruft, und
- man führt eine qualitative und/oder quantitative Analyse des auszutreibenden Produktes durch und bestimmt ein Bestimmungskriterium zur Migration, das ein Verhältnis der Menge des ausgetriebenen Produktes zu der Menge des anfänglich behandelten Körpers bildet.

2. Verfahren nach Anspruch 1, dadurch gekennzeichnet, daß man die Analyse des Teiles des in dem Behälter zurückgewonnenen Produktes durch eine kontinuierliche Spülung des Behälters durchführt.

3. Verfahren nach einem der Ansprüche 1 bis 2, dadurch gekennzeichnet, daß es einen Schritt zur Beaufschlagung des Körpers mit Druck umfaßt, der gegebenenfalls von einem Vorgang zur Aufheizung des Körpers begleitet wird.

4. Verfahren nach einem der Ansprüche 1 bis 3, dadurch gekennzeichnet, daß man die Menge von in einer Gesteinsprobe enthaltenem Kohlenwasserstoff bestimmt.

5. Verfahren nach Anspruch 4, dadurch gekennzeichnet, daß man die Menge des Produktes neben den im Gestein enthaltenen Kohlenwasserstoffen insbesondere an Wasser, Kohlenmonoxid, Kohlendioxid oder Schwefeldioxid bestimmt.

6. Vorrichtung zur Bestimmung der Eignung, die ein Körper aufweist, welcher wenigstens ein aus diesem auszutreibendes Produkt enthält, wobei die Vorrichtung einen Hochdruckraum (1), der selbst ein Einschließungsmilieu umfaßt, und eine Einrichtung zur Erzeugung eines Einschließungsdruckes (2) umfaßt, dadurch gekennzeichnet, daß sie eine Einheit (21) oder einen Probenkörper, die bzw. der im Druckraum vom Einschließungsmilieu hermetisch isoliert ist, umfaßt, wobei die Einheit eine Probe des Körpers und einen Behälter umfaßt.

7. Vorrichtung nach Anspruch 6, dadurch gekennzeichnet, daß sie eine Einrichtung zur Steuerung des zwischen der Probe und dem Behälter vorhandenen Druckgradienten (18) umfaßt.

8. Vorrichtung nach einem der Ansprüche 6 oder 7, dadurch gekennzeichnet, daß die Vorrichtung Einrichtungen zur Spülung (5) des Behälters umfaßt.

9. Vorrichtung nach Anspruch 8, dadurch gekennzeichnet, daß die Einrichtungen zur Spülung Einrichtungen zur Erfassung und/ oder zur Analyse (8, 6, 7) von durch die Spülung in dem Behälter erhaltenen Fluiden umfassen.

10. Vorrichtung nach einem der Ansprüche 8 oder 9, dadurch gekennzeichnet, daß sie Einrichtungen zur Regelung der Temperatur (24, 27) der Spülungsfluide umfaßt.

## Claims

1. A method enabling evaluation of the ability of a body containing a product to expel that product, characterised in that it comprises the following steps:
- the body and a receptacle are positioned adjacent to each other,
- a pressure gradient is produced between the receptacle and the body, this gradient causing at least a part of the product to be expelled to the receptacle, and
- a qualitative and/or quantitative analysis of the product to be expelled is conducted and a migration criterion linked to the ratio of the quantity of the product expelled to the quantity of the initially processed body is determined.

2. A method as claimed in claim 1, characterised in that the part of the product recovered in the receptacle is analyzed by continuous displacement in the receptacle.

3. A method as claimed in one of claims 1 to 2, characterised in that it incorporates a step during which the body is pressurised and possibly heated.

4. A method as claimed in one of claims 1 to 3, characterised in that the quantity of hydrocarbon contained in a rock sample is evaluated.

5. A method as claimed in claim 4, characterised in that the quantity of products other than hydrocarbons contained by the rock is evaluated, in particular the water, carbon monoxide, carbon dioxide or sulphur dioxide.

6. A device enabling evaluation of the ability of a body containing at least one product to expel that product, this device comprising a high pressure chamber (1) itself having a confinement medium, and means for generating a confinement pressure (2), characterised in that it has an assembly (21) or tester hermetically isolated from the confinement medium, this assembly comprising a sample of the body and a receptacle.

7. A device as claimed in claim 6, characterised in that it comprises means for controlling the pressure gradient (18) prevailing between the sample and the receptacle.

8. A device as claimed in one of claims 6 to 7, characterised in that the device has means to provide displacement (5) through the receptacle.

9. A device as claimed in claim 8, characterised in that the displacement means have means for detecting and/or analysing (8, 6, 7) fluids collected by displacement through the receptacle.

10. A device as claimed in one of claims 8 or 9, characterised in that it comprises means for controlling the temperature (24, 27) of the displacement fluids.
